(19) Europäisches Patentamt
European Patent Office
Office européen des brevets

(11) Veröffentlichungsnummer: **0 036 663**
B1

(12) **EUROPÄISCHE PATENTSCHRIFT**

(45) Veröffentlichungstag der Patentschrift: **12.09.84**

(51) Int. Cl.³: **C 07 D 303/42**, C 07 D 301/02, A 61 K 31/335

(21) Anmeldenummer: **81102184.9**

(22) Anmeldetag: **23.03.81**

(54) **Oxiranbuttersäure-Derivate, ihre Herstellung und Verwendung, sowie diese enthaltende Arzneimittel.**

(30) Priorität: **24.03.80 US 133180**

(43) Veröffentlichungstag der Anmeldung: **30.09.81 Patentblatt 81/39**

(45) Bekanntmachung des Hinweises auf die Patenterteilung: **12.09.84 Patentblatt 84/37**

(84) Benannte Vertragsstaaten: **AT BE CH DE FR GB IT LI LU NL SE**

(56) Entgegenhaltungen:
**JOURNAL OF THE AMERICAN CHEMICAL SOCIETY, Band 101, Nr. 22, 24. Oktober 1979 E.J. COREY et al. "Total Synthesis of (+-)-5,6-Oxido-7,9-trans, 11,14-ciseicosapentaenoic Acid, a Possible Precursor of SRSA" Seiten 6748 bis 6749**

(73) Patentinhaber: **F. HOFFMANN-LA ROCHE & CO. Aktiengesellschaft, CH-4002 Basel (CH)**

(72) Erfinder: **Rosenberger, Michael, 79 Arlington Avenue, Caldwell, N.J. (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 036 663 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Oxiranbuttersäure-Derivate der allgemeinen Formel

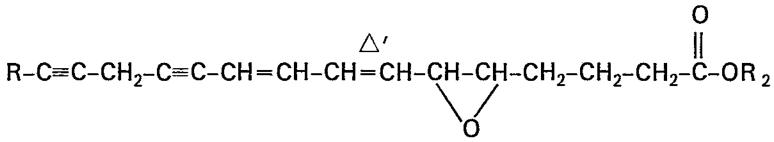

I

worin R die Gruppe $CH_3-CH_2-CH_2-CH_2-CH_2-$ und $R_2$ niederes Alkyl, Aryl oder Aryl-niederes Alkyl bedeuten und das Symbol △′ die trans-Konfiguration der Doppelbindung anzeigt.

Bevorzugt sind Verbindungen der obigen Formel I, worin $R_2$ Methyl, Äthyl, Phenyl oder Benzyl bedeutet. Ebenfalls bevorzugt sind Verbindungen der Formel I, welche bezüglich des Oxiranringes die trans-Konfiguration aufweisen. Eine ganz besonders bevorzugte Verbindung der obigen Formel I ist (R,S)-(all E)-3-(1,3-Tetradekadien-5,8-diyn-1-yl)-1-trans-oxiranbuttersäure-methylester.

Das Material SRS-A (slow reacting substance of anaphylaxis) wird wie Histamin während einer allergischen Reaktion von Säugetierzellen freigesetzt. Das von den Zellen freigesetzte SRS-A bewirkt die Kontraktion der glatten Muskulatur und somit Effekte, wie asthmatische Anfälle. Es bestand somit ein dringendes Bedürfnis nach Arzneimitteln, welche die Wirkungen von SRS-A, das während allergischen Reaktionen von menschlichen Zellen freigesetzt wird, spezifisch antagonisieren.

Herkömmliche antiallergische Mittel, wie Antihistaminika, neutralisieren zwar das während einer allergischen Reaktion freigesetzte Histamin, sind jedoch nicht im Stande, die Wirkungen von SRS-A zu neutralisieren oder zu antagonisieren. Dies hat die Nützlichkeit dieser Antihistaminika als antiasthmatische Mittel eingeschränkt. Deshalb war es seit langer Zeit wünschenswert, ein Mittel zu entwickeln, das die Wirkungen von während einer allergischen Reaktion freigesetztem SRS-A spezifisch antagonisiert.

Bei der Untersuchung von Verbindungen auf ihre Anti-SRS-A-Aktivität wurde natürliches SRS-A verwendet. Eine Schwierigkeit, die bei der Verwendung von aus natürlichen Quellen erhaltenem SRS-A auftaucht, sind die vielen Verunreinigungen, die entfernt werden müssen, bevor das Material zur Bestimmung der spezifischen SRS-A-antagonistischen Wirkung der verschiedenen Substanzen verwendet werden kann. Das aus natürlichen biologischen Quellen stammende SRS-A-Material enthält viele schwierig abzutrennende Verunreinigungen, welche die Bestimmung, ob eine Verbindung spezifisch aktiv ist gegen SRS-A, stören. In einigen Fällen kann ein falsches positives Resultat dadurch erhalten werden, dass eine zu testende Verbindung gegen einige in natürlichem SRS-A-Material enthaltene Komponenten aktiv ist und nicht gegen SRS-A selber. Um solche Komponenten, wie Acetylcholin und Histamin, welche in biologischem SRS-A vorhanden sind, zu neutralisieren, wurden vor Testbeginn verschiedene Zusatzstoffe zum natürlichen SRS-A gegeben. Obwohl diese Zusatzstoffe die obigen Komponenten in einem gewissen Ausmass neutralisieren, können immer noch Rückstände dieser Komponenten vorhanden sein, welche die Versuche stören. Darüber hinaus ist es wünschenswert, die aktiven Komponenten im SRS-A-Material synthetisch herzustellen, um solche Kontaminationen zu vermeiden und um ein Standardmaterial zu erhalten. Ein so erhaltenes Standardmaterial enthält keine Verunreinigungen, welche die Bestimmung der Anti-SRS-A-Eigenschaften von Substanzen stören könnte. Hinzu kommt, dass man durch die Synthese einer SRS-A-aktiven Verbindung die bei der Isolierung von natürlichem SRS-A notwendigen kostspieligen Reinigungstechniken umgehen kann.

Die Oxiranbuttersäure-Derivate der obigen Formel I sind nicht nur wertvolle SRS-A-Antagonisten, sondern auch nützliche Ausgangsstoffe zur Herstellung von SRS-A-aktiven Verbindungen. Sie können hergestellt werden, indem man eine Verbindung der allgemeinen Formel

$$R-C{\equiv}C-CH_2-C{\equiv}C-CH{=}CH-\overset{\triangle'}{CH{=}CH}-CH_2-\overset{\oplus}{\underset{}{S}}(R_5)-R_6\,Y^{\ominus} \qquad II$$

worin Y Halogen bedeutet und $R_5$ und $R_6$ unabhängig voneinander Methyl oder Äthyl bedeuten, oder zusammen mit dem Schwefelatom einen gesättigten 4- bis 6-gliedrigen heterocyclischen Ring bedeuten, wobei das erwähnte Schwefelatom das einzige Heteroatom ist, und △′ obige Bedeutung besitzt, mit einer Verbindung der allgemeinen Formel

$$OCH-CH_2-CH_2-CH_2-\overset{O}{\overset{\|}{C}}-OR_2 \qquad III$$

worin $R_2$ obige Bedeutung besitzt, umsetzt, und

erwünschtenfalls den erhaltenen Stoff in die cis- und trans-Isomeren auftrennt.

Die Verbindung der Formel II wird in Gegenwart einer starken Base mit einer Verbindung der Formel III umgesetzt. Jede herkömmliche starke Base kann für diese Reaktion verwendet werden. Unter den bevorzugten Basen sind Alkalimetall-Basen, wie Natriumhydroxid, Kaliumhydroxid, Lithiumhydroxid, etc. Im allgemeinen kann jede herkömmliche, starke anorganische Base für diese Reaktion verwendet werden. Diese Reaktion wird in einem wässrigen Medium durchgeführt. Temperatur und Druck sind nicht kritisch und man kann demnach bei Raumtemperatur und Atmosphärendruck arbeiten. Andererseits kann man auch bei Temperaturen von etwa −30° bis 45°C arbeiten.

Erwünschtenfalls kann eine erhaltene Verbindung der Formel I unter Verwendung von bekannten Trennmethoden in das cis- und das trans-Epoxid aufgetrennt werden. Jede herkömmliche Trennmethode, wie Flüssigkeits-Chromatographie, kann verwendet werden um diese Auftrennung durchzuführen. Alle in der Flüssigkeits-Chromatographie üblichen Bedingungen können verwendet werden.

Wie bereits weiter oben erwähnt, können die Verbindungen der Formel I als Ausgangsstoffe für die Herstellung von SRS-A-aktiven Verbindungen der allgemeinen Formel

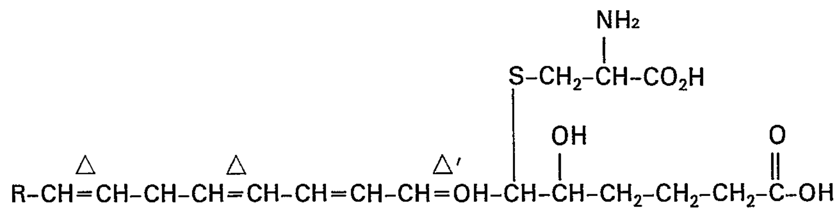

IV

worin das Symbol △ die cis-Konfiguration der entsprechenden Doppelbindung anzeigt und R und △′ obige Bedeutung besitzen, sowie für die Herstellung von SRS-A-aktiven Verbindungen der allgemeinen Formel

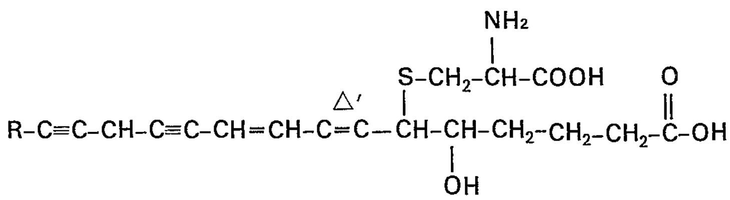

V

worin R und △′ die obige Bedeutung besitzen, und Salzen davon verwendet werden.

Die Verbindungen der obigen Formeln IV und V können bezüglich der 9,10-Doppelbindung in der cis- oder in der trans-Form oder als Gemische dieser beiden Formen vorliegen. Darüber hinaus können die Verbindungen der Formeln IV und V als (5S, 6R)-, (5R, 6S)-, (5S, 6S)- oder (5R, 6R)-Isomere oder als Mischungen dieser Isomeren hergestellt werden.

Aus dem Obigen folgt, dass neue SRS-A-aktive Verbindungen, wie das (5S, 6S)-Isomere der Verbindung der Formel IV, das die Formel

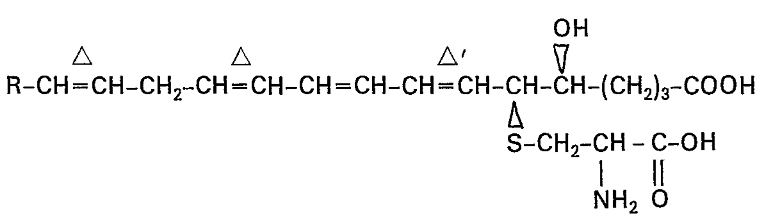

IV-A

worin R, △ und △′ obige Bedeutung besitzen, besitzt, und sein Diastereoisomeres, d.h. das (5R, 6R)-Isomere der Formel

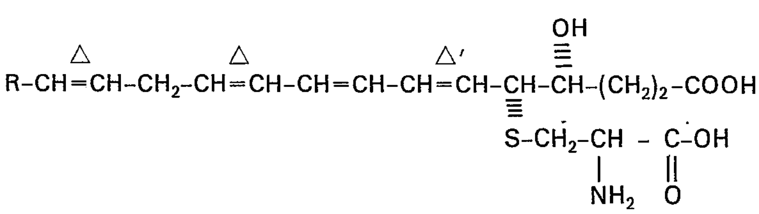

IV-B

worin R, △ und △′ obige Bedeutung besitzen, hergestellt werden können. Auch die Salze von Verbindungen der allgemeinen Formeln IV-A und IV-B können hergestellt werden.

Die Umwandlung von Verbindungen der Formel I in die Verbindungen der Formeln IV und V kann wie folgt durchgeführt werden:

In einem ersten Schritt werden die Verbindungen der obigen Formel I (entweder als cis- oder trans-Epoxide oder als Mischungen davon) in Verbindungen der allgemeinen Formel

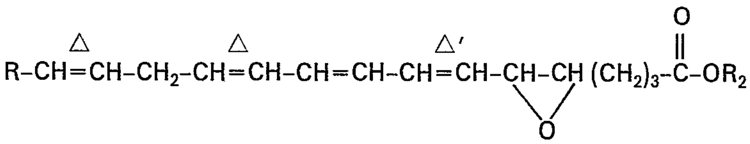

VI

worin R, $R_2$, △ und △′ obige Bedeutung besitzen, übergeführt, und zwar durch Hydrierung in Gegenwart eines selektiven Hydrierungskatalysators. Jeder herkömmliche Katalysator, der die selektive Reduktion einer Dreifachbindung (Acetylen-Bindung) zu einer Doppelbindung erlaubt, kann bei dieser Reaktion verwendet werden. Unter den bevorzugten selektiven Hydrierungskatalysatoren sind die Palladium-Katalysatoren, welche ein desaktivierendes Material, wie Blei, Bleioxid oder Schwefel, enthalten. Unter den bevorzugten selektiven Hydrierungskatalysatoren sind Palladium-Blei-Katalysatoren vom Typus wie sie in Helvetica Chimica Acta 35, 446 (1952) und U.S. Patent No. 2 681 938 beschrieben sind. Diese Katalysatoren sind allgemein bekannt als Lindlar-Katalysatoren. Bei der Durchführung dieser Hydrierung ist die Temperatur nicht kritisch und man kann demnach bei Raumtemperatur und Atmosphärendruck arbeiten. Andererseits kann man auch bei höheren oder tieferen Temperaturen arbeiten. Im allgemeinen wird diese Reaktion in einem inerten organischen Lösungsmittel durchgeführt. Jedes herkömmliche inerte organische Lösungsmittel kann dabei verwendet werden, z.B. Essigester, Toluol, Petroläther oder Hexan. Die Hydrierung einer Verbindung der Formel I unter Verwendung eines selektiven Hydrierungskatalysators liefert eine Doppelbindung mit einer cis-Konfiguration. Daraus folgt, dass die selektive Hydrierung einer Verbindung der Formel I, welche zwei Acetylen-Bindungen enthält, eine Verbindung mit zwei Doppelbindungen liefert, die beide die cis-Konfiguration aufweisen.

Die Verbindungen der allgemeinen Formel VI (entweder als cis- oder trans-Epoxid oder als Mischungen davon) können anschliessend in Verbindungen der allgemeinen Formel

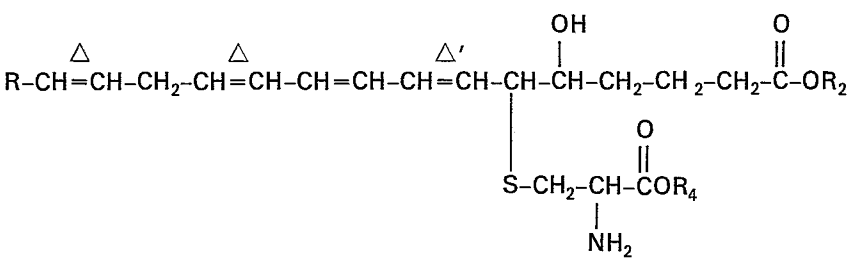

VII

worin $R_4$ Wasserstoff bedeutet oder zusammen mit der Gruppe -COO- eine hydrolysierbare Estergruppe bedeutet, und R, $R_2$, △ und △′ obige Bedeutung besitzen, übergeführt werden, und zwar durch Reaktion mit einem Cystein-Derivat der allgemeinen Formel

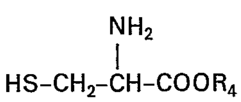

VIII

worin $R_4$ obige Bedeutung besitzt.

Die Reaktion einer Verbindung der Formel VI mit einer Verbindung der Formel VIII kann in Gegenwart einer organischen Base durchgeführt werden. Jede herkömmliche organische Base kann für diese Reaktion verwendet werden. Unter den bevorzugten Basen sind die tertiären Amine, wie die Tri(nieder Alkyl)amine und die cyclischen Amine. Unter den besonders bevorzugten Basen sind Triäthylamin, Pyridin, etc. Weitere Amine, welche verwendet werden können, sind Monoäthyl-dimethyl-amin, Tri(isopropyl)amin, etc. Diese Reaktion wird vorzugsweise in einem Gemisch aus Wasser und einem organischen Lösungsmittel durchgeführt. Als organisches Lösungsmittel kann jedes herkömmliche mit Wasser mischbare organische Lösungsmittel, wie die weiter unten aufgeführten, verwendet werden. Temperatur und Druck sind nicht kritisch und man kann demnach bei Raumtemperatur und Atmosphärendruck arbeiten. Andererseits kann man auch bei höheren oder niedrigeren Temperaturen arbeiten. Im allgemeinen arbeitet man in einem Temperaturbereich von etwa 0°C bis 50°C.

Wenn die Verbindung der Formel VI das trans-Epoxid ist und die Verbindung der Formel VIII die L- oder D-Konfiguration aufweist, so erhält man die Verbindung der Formel VII als Mischung der (5R, 6S)- und (5S, 6R)-Isomeren. Erwünschtenfalls kann das Gemisch dieser Isomeren unter Verwendung von herkömmlichen Methoden aufgetrennt werden. Vorzugsweise verwendet man dabei die Flüssigkeits-Chromatographie. Jede der in der Flüssigkeits-Chromatographie üblichen Bedingungen können für die vorliegende Trennung verwendet werden. Verwendet man hingegen das entsprechende cis-Epoxid und die

Verbindung der Formel VIII in der L- oder D-Konfiguration, so erhält man die Verbindung der Formel VII als Mischung der (5S, 6S)- und (5R, 6R)-Isomeren. Diese Mischung kann wie weiter oben beschrieben mittels Flüssigkeits-Chromatographie aufgetrennt werden. Verwendet man andererseits die Verbindung der Formel VI als Mischung der cis- oder trans-Epoxide und die Verbindung der Formel VIII in racemischer Form, so erhält man die Verbindung der Formel VII als Mischung aller möglichen isomeren Formen.

Die Verbindungen der allgemeinen Formel VII können anschliessend durch Hydrolyse in Verbindungen der allgemeinen Formel IV übergeführt werden. Jede herkömmliche Hydrolysemethode kann dabei verwendet werden, beispielsweise kann man eine Verbindung der Formel VII mit einer wässrigen Alkalimetall-Base behandeln. Bedeuten $R_2$ und $R_4$ eine Estergruppe, so wird zuerst die Estergruppe $R_2$ hydrolysiert, so dass man eine entsprechende Verbindung der Formel VII erhält, worin aber $R_2$ Wasserstoff und $R_4$ eine Estergruppe bedeuten. Durch weitere Hydrolyse wird dann auch die Gruppe $R_4$ entfernt. Ist das (5S, 6R)-Isomere der Formel IV erwünscht, so wird die Verbindung der Formel VII in das (5S, 6R)- und das (5R, 6S)-Isomere aufgetrennt, und das (5S, 6R)-Isomere hydrolysiert. Andererseits erhält man durch Hydrolyse des (5R, 6S)-Isomeren der Verbindung der Formel VII das (5R, 6S)-Isomere der Verbindung der Formel IV.

Die Verbindungen der Formel V können hergestellt werden, indem man eine Verbindung der Formel I mit einer Verbindung der Formel VIII umsetzt, und zwar in Analogie zur weiter oben beschriebenen Reaktion einer Verbindung der Formel VI mit einer Verbindung der Formel VIII zu einer Verbindung der Formel VII. Durch eine solche Reaktion erhält man eine Verbindung der allgemeinen Formel

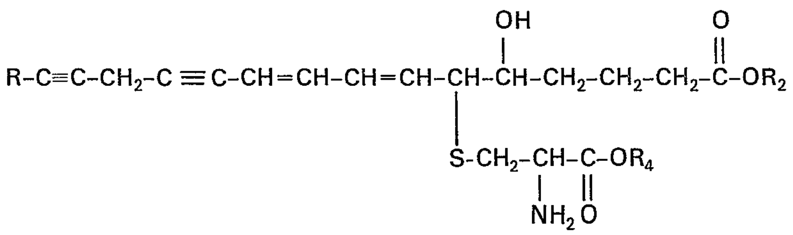

IX

worin R, $R_2$ und $R_4$ obige Bedeutung besitzen.

Die Estergruppe in einer Verbindung der obigen Formel IX kann durch herkömmliche Verseifungsmethoden wie weiter oben beschrieben hydrolysiert werden.

Die Verbindungen der Formeln IV und V können als basische Salze verwendet werden. Erfindungsgemäss kann jedes pharmazeutisch annehmbare Salz verwendet werden. Unter den bevorzugten pharmazeutisch annehmbaren Salzen sind die Alkalimetall-Salze, wie die Lithium-, Natrium- und Kaliumsalze, die Erdalkalimetall-Salze, wie die Calciumsalze, Salze mit Aminen, wie mit niederen Alkylaminen, z.B. Äthylamin, und Salze mit hydroxy-substituierten niederen Alkylaminen. Bevorzugt sind auch die Ammoniumsalze. Es kommen auch Salze mit organischen Basen und mit Aminen in Frage, wie z.B. Salze mit N-Äthylpiperidin, Procain, Dibenzylamin, N-Dibenzyläthyläthylendiamin, Alkylaminen oder Dialkylaminen und Salze mit Aminosäuren (z.B. Salze mit Arginin und Glycin). Die Ausgangsstoffe der Formel II sind neu und können hergestellt werden, indem man eine Verbindung der allgemeinen Formel

$$R-C\equiv C-CH_2-Y \qquad X$$

worin R und Y obige Bedeutung besitzen, mit einer Verbindung der allgemeinen Formel

$$HC\equiv C-CH=CH-CH_2-OR_1 \qquad XI$$

worin $R_1$ zusammen mit dem Sauerstoffatom eine Ätherschutzgruppe bedeutet, welche nach Hydrolyse die freie Hydroxygruppe liefert, zu einer Verbindung der allgemeinen Formel

$$R-C\equiv C-CH_2-C\equiv C-CH=CH-CH_2-OR_1 \qquad XII$$

worin R und $R_1$ obige Bedeutung besitzen, umsetzt.

Die Verbindung der Formel X wird über eine Grignard-Reaktion mit einer Verbindung der Formel XI umgesetzt. Es werden dabei die bei Grignard-Reaktionen üblichen Bedingungen verwendet.

Eine erhaltene Verbindung der Formel XII wird über die folgenden Zwischenprodukte in eine Verbindung der Formel II übergeführt:

$$R-C\equiv C-CH_2-C\equiv C-CH=CH-CH_2OH \qquad XIII$$

$$R-C\equiv C-CH_2-C\equiv C-CH=CH-CHO \qquad XIV$$

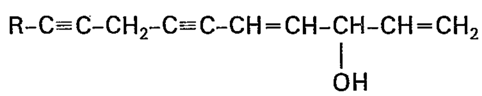

XV

$$\overset{\triangle'}{R-C\equiv C-CH_2-C\equiv C-CH=CH-CH=CH-CH_2Y} \qquad XVI$$

Eine Verbindung der Formel XII wird durch Ätherhydrolyse in eine Verbindung der Formel XIII übergeführt. Diese Ätherhydrolyse kann nach an sich bekannten Methoden durchgeführt werden. Beispielsweise kann man eine Verbindung der Formel XII mit einer Säure unter wässrigen Bedingungen behandeln. Geeignete Säuren sind z.B. anorganische Säuren, wie Schwefelsäure,

Salzsäure, usw. Temperatur und Druck sind nicht kritisch und man kann demnach bei Raumtemperatur und Atmosphärendruck arbeiten. Erwünschtenfalls kann man auch bei höheren oder tieferen Temperaturen arbeiten.

Durch Behandeln einer Verbindung der Formel XIII mit einem Oxydationsmittel erhält man eine Verbindung der Formel XIV. Jedes herkömmliche, für die Oxydation von Alkoholen zu Aldehyden geeignete Oxydationsmittel kann verwendet werden. Bevorzugte Oxydationsmittel sind beispielsweise Silbersalze, wie Silbercarbonat, Mangandioxid, Jones-Reagens, chromhaltige Oxydationsmittel, wie Pyridinium-dichromat, und dergleichen. Es werden dabei die bei solchen Oxydationsmitteln üblichen Reaktionsbedingungen angewendet.

Durch Reaktion einer Verbindung der Formel XIV mit einem Vinyl-Magnesiumhalogenid in einer Grignard-Reaktion erhält man eine Verbindung der Formel XV. Man verwendet dabei die bei solchen Grignard-Reaktionen üblichen Reaktionsbedingungen. Durch Behandeln einer Verbindung der Formel XV mit einem Halogenierungsmittel erhält man eine Verbindung der Formel XVI. Jedes herkömmliche Halogenierungsmittel kann dabei verwendet werden. Bevorzugte Halogenierungsmittel sind beispielsweise Phosphortrihalogenide, wie Phosphortribromid, Phosphortrichlorid, und dergleichen. Man verwendet dabei die bei solchen Halogenierungsmitteln üblichen Reaktionsbedingungen. Die durch die Halogenierung einer Verbindung der Formel XV bewirkte Umlagerung der Doppelbindung führt zu einer Verbindung der Formel XVI, worin die Doppelbindung die trans-Konfiguration aufweist. Bei der Umwandlung einer Verbindung der Formel XVI in eine Verbindung der Formel I wird die trans-Konfiguration dieser Doppelbindung beibehalten.

Durch Reaktion einer Verbindung der Formel XVI mit einem Sulfid der Formel

$R_5$-S-$R_6$ XVII

worin $R_5$ und $R_6$ obige Bedeutung besitzen, erhält man ein Salz der Formel II.

Als Verbindung der Formel XVII verwendet man vorzugsweise Diäthylsulfid, Dimethylsulfid, Tetrahydrothiophen, Thietan und Tetrahydrothiopyran. Im allgemeinen führt man diese Reaktion in einer Mischung aus Wasser und einem organischen Lösungsmittel durch. Als organisches Lösungsmittel kann man jedes herkömmliche mit Wasser mischbare organische Lösungsmittel verwenden. Geeignete organische Lösungsmittel sind beispielsweise Alkohole, wie Methanol, Äthanol, Isopropanol und dergleichen, Äther, wie Tetrahydrofuran und dergleichen. Temperatur und Druck sind nicht kritisch; vorzugsweise arbeitet man bei Raumtemperatur und Atmosphärendruck. Man kann aber auch in einem Temperaturbereich von 0°C bis 45°C arbeiten.

Die Verbindungen der Formel XI können ausgehend von Epoxiden der allgemeinen Formel

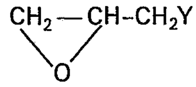 XVIII

worin Y obige Bedeutung besitzt, hergestellt werden. Durch Umsetzen eines Epoxides der obigen allgemeinen Formel XVIII mit einem Alkalimetall-Acetylid, wie Natrium-Acetylid, erhält man eine Verbindung der allgemeinen Formel

$\triangle'$
CH≡C-CH=CH-$CH_2$OH XIX

worin $\triangle'$ obige Bedeutung besitzt.

Man verwendet dabei die bei solchen Reaktionen üblichen Reaktionsbedingungen. Bei der Reaktion eines Halogenids der Formel XVIII mit einem Alkalimetall-Acetylid erhält man eine Verbindung der Formel XIX, worin die Doppelbindung die trans-Konfiguration aufweist. Andererseits ist es ohne weiteres möglich, entweder das cis- oder das trans-Isomere einer Verbindung der Formel XIX oder eine Mischung dieser beiden Formeln herzustellen, und zwar dadurch, dass man zuerst Natrium-Acetylid mit dem Aldehyd der Formel

$CH_2$=CH-CHO XX

umsetzt und so eine Verbindung der Formel

CH≡C-CH-CH=CH
|
OH XXI

erhält. Man verwendet dabei die bei solchen Reaktionen üblichen Reaktionsbedingungen. Die erhaltene Verbindung der Formel XXI wird anschliessend einer säurekatalysierten Umlagerung unterworfen und liefert eine Verbindung der Formel XIX als Mischung der cis- und trans-Isomeren. Erwünschtenfalls kann man diese Isomeren nach an sich bekannten Methoden trennen, beispielsweise durch Chromatographie. Bei der Herstellung von Verbindungen der Formeln I, IV und V aus der Verbindung der Formel XIX erhält man Verbindungen, worin die 9,10-Doppelbindung dieselbe Konfiguration aufweist wie die Doppelbindung in der Verbindung der Formel XIX.

Die Verbindung der Formel XIX kann nach an sich bekannten Methoden veräthert werden. Jedes herkömmliche Verätherungsmittel, wie Äthyl-vinyl-äther, und dergleichen, kann dabei verwendet werden. Man wendet dabei die in solchen Fällen üblichen Reaktionsbedingungen an.

Die Verbindung der Formel X kann durch Behandeln von 2-Octyn-1-ol mit einem Halogenierungsmittel hergestellt werden.

Die Verbindungen der Formeln III, VIII und XVII sind bekannt oder können in Analogie zur Herstellung der bekannten Verbindungen hergestellt werden.

Der in der vorliegenden Beschreibung verwendete Ausdruck «Halogen» bezeichnet Fluor, Chlor, Brom und Jod, wobei Chlor und Brom bevorzugt sind. Der Ausdruck «hydrolysierbare Äthergruppe» bezeichnet jede herkömmliche hydrolysierbare Äthergruppe. Bevorzugte hydrolysierbare Äther sind die α-nieder Alkoxy-nieder Alkyl-, Tetrahydropyranyl-, Benzyl-, Benzhydryl-, Trityl-, t-Butyl- und 4-Methyl-5,6-dihydro-2H-pyranyl-äther. Bevorzugte nieder Alkoxy-nieder Alkyl-äther sind Methoxymethyl-äther, α-Methoxyäthyl-äther, α-Äthoxy-äthyl-äther und dergleichen.

Der Ausdruck «niederes Alkyl» bezeichnet Alkylgruppen mit 1-7 Kohlenstoffatomen, wie Methyl, Äthyl, Propyl, Butyl und dergleichen. Der Ausdruck «niederes Alkoxy» bezeichnet niedere Alkoxygruppen mit 1-7 Kohlenstoffatomen, wie Methoxy, Äthoxy, Isopropoxy und dergleichen.

Der in der vorliegenden Beschreibung verwendete Ausdruck «hydrolysierbare Estergruppe» bezeichnet eine herkömmliche Estergruppe, welche durch übliche Hydrolysemethoden in die freie Säure übergeführt werden kann. Geeignete Ester sind beispielsweise die niederen Alkylester, insbesondere die Methyl- und die Äthylester, Arylester, insbesondere die Phenylester und die Arylnieder-alkylester, insbesondere die Benzylester.

Der Ausdruck «Aryl» bezeichnet Phenyl, welche unsubstituiert sein können oder durch niederes Alkyl oder Nitro substituiert sein können, und Naphthyl und Anthryl, welche unsubstituiert sein können oder durch eine der oben genannten Gruppen substituiert sein können. Phenyl ist die bevorzugte Arylgruppe.

Wie weiter oben bereits erwähnt, sind die Verbindungen der allgemeinen Formel I Antagonisten von SRS-A und sind demnach nützlich bei der Behandlung von allergischen Reaktionen, insbesondere bei der Behandlung von Asthma bronchiale.

Prophylaktisch wirksame Mengen einer Verbindung der allgemeinen Formel I oder pharmazeutische Präparate, enthaltend eine prophylaktisch wirksame Menge einer solchen Verbindung, können nach an sich bekannten Methoden verabreicht werden. Diese Substanzen können als Einzelsubstanzen verabreicht werden oder zusammen mit anderen Wirkstoffen, z.B. mit Antagonisten oder Vermittlern von Anaphylaxie, wie Antihistaminika, oder mit antiasthmatisch wirksamen Steroiden, wie Prednison und Prednisolon. Diese Substanzen können oral, parenteral oder durch Inhalation, z.B. in Form eines Aerosols, mikropulverisierten Pulvers oder in Form einer versprühten Lösung, verabreicht werden. Für die orale Verabreichung eignen sich Pillen, Tabletten, Kapseln, z.B. in Mischungen mit Talk, Stärke, Milchzucker oder anderen inerten Trägermaterialien, d.h. pharmazeutisch annehmbare Träger, oder wässrige Lösungen, Suspensionen, verkapselte Suspensionen, Gele, Elixiere oder wässrige alkoholische Lösungen, z.B. in Mischungen mit Zukker oder anderen Süssstoffen, Aromastoffen, Färbemitteln, Verdickungsmitteln und anderen herkömmlichen pharmazeutischen Excipientien. Für die parenterale Verabreichung eignen sich Lösungen oder Suspensionen, z.B. wässrige Lösungen oder Erdnussöl-Lösungen oder Suspensionen unter Verwendung von üblichen Excipientien und Trägermaterialien. Für die Verabreichung in Form von Aerosolen können diese Substanzen in einem geeigneten pharmazeutisch annehmbaren Lösungsmittel gelöst werden, z.B. in Äthanol oder Wasser oder in Kombinationen von mischbaren Lösungsmitteln, und mit einem pharmazeutisch annehmbaren Treibgas vermischt werden. Solche Aerosole werden in geeignete Druckflaschen abgefüllt, welche mit einem für solche Zwecke geeigneten Ventil versehen sind. Dieses Ventil ist vorzugsweise ein Dosierungsventil, d.h. ein Ventil, das bei Betätigung eine vorbestimmte Menge einer wirksamen Dosis des Aerosols freigibt.

Die Dosierung der zu verabreichenden Verbindung der allgemeinen Formel I und die Häufigkeit der Verabreichung hängen ab von der Wirksamkeit und der Länge der Wirkung dieser Verbindung, von der Art der Verabreichung, sowie von der Stärke der zu behandelnden Krankheit, vom Alter des zu behandelnden Individuums usw. Im allgemeinen dürfte eine tägliche Dosis von 0,01 bis etwa 100 mg/kg Körpergewicht, vorzugsweise 0,1 bis etwa 10 mg/kg Körpergewicht, angemessen sein, wobei die Verabreichung in einer einzigen Dosierung oder in mehreren Dosen erfolgen kann.

Die folgenden Beispiele und Experimente illustrieren die vorliegende Erfindung. Alle Temperaturen sind in Celsiusgraden angegeben. Der verwendete Äther ist Diäthyläther. Für die Flüssigkeits-Chromatographie wurde ein handelsüblicher Hochdruck-Flüssigkeits-Chromatograph und zwei Kieselgelkolonnen verwendet. Die Durchflussrate betrug jeweils 250 ml/Minute.

Beispiel 1

1,5 l flüssiges Ammoniak werden mit Acetylen gesättigt und unter Durchleiten von Acetylen portionenweise (1 g) mit insgesamt 50,6 g Natrium behandelt. Die farblose Mischung wird anschliessend bei −45° über einen Zeitraum von 1,5 Stunden mit 92,5 g Epichlorhydrin behandelt. Man rührt während 1,5 Stunden bei −45° und während weiteren 3 Stunden bei der Rückflusstemperatur und versetzt anschliessend mit 75 g Ammoniumchlorid. Man lässt über Nacht das Ammoniak abdampfen, nimmt den Rückstand in 500 ml Wasser auf und extrahiert zehnmal mit je 300 ml Äther. Die vereinigten Ätherauszüge werden über Magnesiumsulfat getrocknet und zur Trockne eingedampft. Der Rückstand wird unter Nachspülen mit Hexan/Essigester (1:1) über 80 g Kieselgel filtriert. Nach Entfernen des Lösungsmittels und Reinigung des Rückstandes durch Flüssigkeits-Chromatographie [unter Eluieren mit Hexan/Essigester (4:1)] erhält man 2-trans-Penten-4-yn-1-ol.

Beispiel 2

47,7 g 2-trans-Penten-4-yn-1-ol werden in 50 ml Äther aufgelöst, auf 0° abgekühlt und mit 50 ml Äthylvinyl-äther behandelt. Man versetzt diese

Lösung mit 0,1 g fester p-Toluolsulfonsäure, wobei eine leicht exotherme Reaktion stattfindet. Man rührt noch während 20 Minuten und versetzt anschliessend mit 2 ml Triäthylamin. Nach Eindampfen im Vakuum wird der Rückstand in Äther aufgenommen, mit wässriger Natriumbicarbonatlösung gewaschen, über Magnesiumsulfat getrocknet und eingedampft. Der Rückstand wird durch Flüssigkeits-Chromatographie [unter Eluieren mit Hexan/Essigester (6:1)] und Destillation (Siedepunkt 88–98°, 26 mm Hg) gereinigt. Man erhält (E)-4-Methyl-3,5-dioxa-7-decen-9-yn.

Beispiel 3

Eine Lösung von 37,8 g 2-Octyn-1-ol in 150 ml Äther, das 3 ml Pyridin enthält, wird auf −35° abgekühlt und über einen Zeitraum von 45 Minuten mit einer Lösung von 28,9 g Phosphortribromid behandelt. Die Mischung wird anschliessend während 2 Stunden bei −30°, während 3 Stunden bei Raumtemperatur und während 0,5 Stunden bei 40° gerührt. Die Reaktionsmischung wird anschliessend auf Raumtemperatur abgekühlt, auf Eis gegossen und mit Äther extrahiert. Die vereinigten Ätherauszüge werden mit wässriger Natriumbicarbonatlösung und halbgesättigter Kochsalzlösung gewaschen, über Magnesiumsulfat getrocknet und eingedampft. Nach Destillation des Rückstandes erhält man 1-Brom-2-octyn vom Siedepunkt 106–110°, 25 mm Hg.

Beispiel 4

Eine Lösung von Äthyl-magnesiumbromid in Tetrahydrofuran (47 ml, 1,17 M) wird tropfenweise mit einer Lösung von 7,7 g (E)-4-Methyl-3,5-dioxa-7-decen-9-yn in 10 ml Tetrahydrofuran behandelt. Nach beendeter Zugabe wird die Mischung während 45 Minuten auf 60° erwärmt, auf Raumtemperatur abgekühlt, mit 0,35 g wasserfreiem Kupfer (I)chlorid (während 16 Stunden bei 145°, 0,05 mm Hg getrocknet) versetzt und während 10 Minuten gerührt. Die erhaltene Mischung versetzt man tropfenweise mit 7,23 g 1-Brom-2-octyn in 10 ml Tetrahydrofuran (schwache exotherme Reaktion) und erwärmt anschliessend während 45 Minuten auf 60°. Danach kühlt man auf Raumtemperatur, giesst auf gesättigte wässrige Ammoniumchloridlösung und extrahiert mit Äther. Die vereinigten Auszüge werden mit halbgesättigter Kochsalzlösung gewaschen, über Magnesiumsulfat getrocknet und eingedampft. Der Rückstand wird durch Flüssigkeits-Chromatographie [unter Eluieren mit Hexan, das 3% Essigester enthält (v/v)] gereinigt. Man erhält (E)-4-Methyl-3,5-dioxa-7-octadecen-9,12-diyn.

Beispiel 5

Eine Lösung von 3,76 g (E)-4-Methyl-3,5-dioxa-7-octadecen-9,12-diyn in 60 ml Aceton wird mit 6 ml 0,2N-wässriger Schwefelsäure versetzt und während 3 Stunden bei Raumtemperatur stehengelassen. Die Hälfte des Acetons wird im Vakuum entfernt und der Rückstand auf Eiswasser gegossen. Man extrahiert mit Äther. Die vereinigten Auszüge werden über Magnesiumsulfat getrocknet und eingedampft. Der Rückstand wird durch Flüssigkeits-Chromatographie unter Eluieren mit Hexan/Essigester (4:1) gereinigt. Man erhält reines (E)-2-Tridecen-4,7-diyn-1-ol.

Beispiel 6

60 g Pyrridinium-dichromat werden zusammen mit 250 ml Methylenchlorid bei 0° gerührt und über einen Zeitraum von 5 Minuten mit einer Lösung von 20 g (E)-2-tridecen-4,7-diyn-1-ol in Methylenchlorid behandelt. Man entfernt das Kühlbad und rührt die Reaktionsmischung während weiteren 3 Stunden (schwache exotherme Reaktion). Man versetzt mit 250 ml Äther. Das feste Material wird abfiltriert und mit Äther gewaschen. Nach Eindampfen der vereinigten Filtrate wird der Rückstand mit Hexan behandelt und über Kiselgel filtriert. Das Filtrat wird anschliessend fünfmal mit je 250 ml Wasser gewaschen, über Magnesiumsulfat getrocknet und auf 100 ml eingeengt. Aus dieser Lösung erhält man mittels Flüssigkeits-Chromatographie unter Eluieren mit Hexan/Essigester (4:1) reines (E)-2-Tridecen-4,7-diyn-1-al.

Beispiel 7

150 ml Tetrahydrofuran werden mit 35 ml einer 2,2N-Lösung von Vinyl-magnesiumchlorid in Tetrahydrofuran versetzt, auf −60° abgekühlt und über einen Zeitraum von 5–10 Minuten mit einer Lösung von 12,6 g (E)-2-Tridecen-4,7-diyn-1-al in 50 ml Tetrahydrofuran versetzt. Man entfernt das Kühlbad und lässt auf 0° erwärmen. Anschliessend versetzt man mit 200 ml Äther und mit 10 ml einer gesättigten wässrigen Ammoniumchloridlösung. Man rührt die Mischung während 15 Minuten, versetzt mit Magnesiumsulfat und rührt noch während 30 Minuten. Das feste Material wird abfiltriert und mit Äther gewaschen. Nach Eindampfen der vereinigten Filtrate wird der Rückstand durch Flüssigkeits-Chromatographie unter Eluieren mit Essigester/Hexan (1:9) gereinigt. Man erhält reines (E)-1,4-Pentadecadien-6,9-diyn-3-ol.

Beispiel 8

Eine Lösung von 8,5 g (E)-1,4-Pentadecadien-6,9-diyn-3-ol in 200 ml Äther wird auf −40° abgekühlt und über einen Zeitraum von 5 Minuten mit 3,7 ml Phosphortribromid in 70 ml Äther behandelt. Man entfernt das Kühlbad und lässt während 1 Stunde auf 0° erwärmen. Man giesst auf Eis und extrahiert mit Äther. Die Ätherauszüge werden mit Wasser und mit 10 ml Natriumcarbonatlösung gewaschen, über Magnesiumsulfat getrocknet und eingedampft. Der Rückstand wird durch Flüssigkeits-Chromatographie unter Eluieren mit Hexan gereinigt. Man erhält (E,E)-1-Brom-2,4-pentadecadien-6,9-diyn als reines Material.

Beispiel 9

13,8 g (E,E)-1-Brom-2,4-pentadecadien-6,9-diyn werden in 14 ml Tetrahydrothiophen gelöst und anschliessend mit 30 ml wässrigem Methanol (9:1) versetzt. Das Zweiphasensystem wird während 40 Minuten bei Raumtemperatur gerührt und anschliessend während 10 Minuten bei 40°, 20 mm Hg und bei Raumtemperatur, 0,5 mm Hg eingedampft. Man erhält (E,E)-2,4-Pentadecadien-6,9-diyn-1-yl-tetrahydrothiophenium-bromid als teilweise festen Stoff.

Beispiel 10

24,2 g (E,E)-2,4-Pentadecadien-6,9-diyn-1-yl-tetrahydrothiophenium-bromid werden in 100 ml Methylenchlorid, das 0,5 g Benzyltriäthyl-ammoniumchlorid und 9,6 g (1,5 Mol) Methyl-4-formylbutyrat enthält, aufgelöst und in einem Trockeneis/Acetonbad auf −30° abgekühlt. Man versetzt rasch (10 Sekunden) mit 75 ml kalter (−5°) 10N-Natronlauge und rührt gut während 60 Sekunden. Man stellt den Rührer ab und friert die wässrige Phase rasch ein. Nach Abdekantieren der organischen Phase wird der Rückstand dreimal mit je 100 ml Äther gewaschen (auftauen und einfrieren). Die vereinigten organischen Auszüge werden anschliessend mit Wasser gewaschen, über Magnesiumsulfat getrocknet und eingedampft. Die rohe Epoxidmischung wird durch Flüssigkeits-Chromatographie unter Eluieren mit Essigester/Hexan (1:9) gereinigt. Man erhält reines trans-Epoxid, d.h. (R,S)-(all E)-3-(1,3-Tetradecadien-5,8-diyn-1-yl)-1-trans-oxiranbuttersäuremethylester, eine Mischfraktion, enthaltend das cis- und das trans-Isomere, und das reine cis-Epoxid, d.h. (R,S)-(all E)-3-(1,3-tetradecadien-5,8-diyn-1-yl)-cis-oxiranbuttersäure-methylester.

Für die obige Trennung wurden die beiden Kieselgelsäulen durch Waschen mit 1 l Essigsäure/Essigester (1:9), 500 ml Methanol, 1 l Triäthylamin/Essigester (1:9), 500 ml Methanol, 1 l Aceton, 1 l Essigester und 1 l Hexan vorkonditioniert.

Beispiel 11

1 g (R,S)-(all E)-3-(1,3-Tetradecadien-5,8-diyn-1-yl)-1-trans-oxiranbuttersäure-methylester wird in 20 ml Hexan aufgelöst, wobei man eine trübe Lösung erhält. Man versetzt mit 1 g Lindlar-Katalysator (hergestellt wie in Organic Synthesis, Collective Vol. V, Seiten 880–883 beschrieben) und filtriert die Mischung über Diatomenerde. Man gibt noch 1 g des Katalysators hinzu und hydriert die Mischung bei Raumtemperatur und Atmosphärendruck. Nach Aufnahme von 175 ml Wasserstoff werden die Feststoffe abfiltriert und das Filtrat eingedampft. Der Rückstand wird durch Flüssigkeits-Chromatographie unter Eluieren mit Hexan/Essigester, das 2% Triäthylamin enthält, (9:1) gereinigt. Man erhält (R,S)-(E,E,Z,Z)-3-(1,3,5,8-Tetradecatetraen-1-yl)-1-trans-oxiranbuttersäure-methylester.

Beispiel 12

Der pH einer Lösung von 2,5 g L-Cystein-methylester-hydrochlorid in 35 ml wässrigem Methanol (30:5) wird mit Triäthylamin auf 8,5 eingestellt. Diese Lösung gibt man anschliessend zu 2,5 g (R,S)-(E,E,Z,Z)-3-(1,3,5,8-Tetradecatetraen-1-yl)-1-trans-oxiranbuttersäure-methylester und rührt die erhaltene Mischung während 4,5 Stunden bei Raumtemperatur. Nach Eindampfen im Vakuum wird der Rückstand zwischen Äther und Wasser verteilt. Die ätherische Phase wird mit Wasser und mit halbgesättigter Kochsalzlösung gewaschen, über Magnesiumsulfat getrocknet und eingedampft. Durch Reinigung des Rückstandes mittels Flüssigkeits-Chromatographie unter Eluieren mit Essigester liefert als erstes eluiertes Material das (5R, 6S)-Isomere, d.h. (5R, 6S)-(E,E,Z,Z)-S-(5-Hydroxy-1-methoxy-1-oxo-eicosa-7,9,11,14-tetraen-6-yl)-L-cystein-methylester. Ausserdem erhält man noch eine Mischung von Diastereoisomeren, das vorwiegend das (5S, 6R)-Isomere enthält, und das (5S, 6R)-Isomere, d.h. (5S, 6R)-(E,E,Z,Z)-S-(5-Hydroxy-1-methoxy-1-oxo-eicosa-7,9,11,14-tetraen-6-yl)-L-cystein-methylester.

Beispiel 13

Eine Lösung von 0,3 g (5S, 6R)-(E,E,Z,Z)-S-(5-Hydroxy-1-methoxy-1-oxo-eicosa-7,9,11,14-tetraen-6-yl)-L-cystein-methylester in 15 ml Methanol wird mit einer Lösung von 150 mg Kaliumhydroxid in 1,5 ml Wasser versetzt und während 90 Minuten bei Raumtemperatur stehengelassen. Man versetzt anschliessend mit 20 ml Wasser und engt im Vakuum ein. Die erhaltene wässrige Lösung wird an einer mit einem Ionenaustauscher ($H^+$-Form) beladenen Kolonne chromatographiert. Man eluiert bis zur neutralen Reaktion mit Wasser und anschliessend mit 10%igen wässrigem Ammoniak. Sobald Ammoniak im Eluat erscheint, sammelt man 150 ml, versetzt mit 10 ml n-Butanol und dampft bei 45°, 20 mm Hg ein, bis das n-Butanol und Ammoniak entfernt sind. Die verbleibende wässrige Lösung wird anschliessend gefriergetrocknet, wobei man (5S, 6R)-(E,E,Z,Z)-S-(5-Hydroxy-1-hydroxy-1-oxo-eicosa-7,9,11,14-tetraen-6-yl)-L-cystein-mono-ammoniumsalz als Pulver erhält.

Beispiel 14

Durch Hydrolyse von (5R, 6S)-(E,E,Z,Z)-S-(5-Hydroxy-1-methoxy-1-oxo-eicosa-7,9,11,14-tetraen-6-yl)-L-cystein-methylester, in Analogie zu den Angaben im Beispiel 13, erhält man (5R, 6S)-(E,E,Z,Z)-S-(5-hydroxy-1-hydroxy-1-oxo-eicosa-7,9,11,14-tetraen-6-yl)-L-cystein-mono-ammoniumsalz.

Beispiel 15

Der pH einer Lösung von 1,2 g (L)-Cystein-methylester-hydrochlorid in 20 ml wässrigem Methanol (4:1) wird mit 3 ml Triäthylamin auf 8,5 eingestellt, zu 1,8 g (R,S)-(all E)-3-(1,3-tetradecadien-5,8-diyn-1-yl)-trans-oxiranbuttersäuremethylester gegeben und über Nacht bei Raumtemperatur gerührt. Man entfernt das meiste Methanol im Vakuum und verteilt den Rückstand zwischen Äther und Wasser. Die ätherischen Phasen werden mit Wasser gewaschen, vereinigt, über Magnesiumsulfat getrocknet und eingedampft. Die erhaltene rohe Mischung wird

durch Flüssigkeits-Chromatographie gereinigt. Man erhält (5S, 6R)-(E,E)-S-(5-Hydroxy-1-methoxy-1-oxo-eicosa-7,9-dien-11,14-diyn-6-yl)-L-cystein-methylester und das entsprechende Diastereoisomere (5R, 6S)-S-(5-Hydroxy-1-methoxy-1-oxo-eicosa-7,9-dien-11,14-diyn-6-yl)-L-cystein.

Beispiel 16

Eine Lösung von 0,45 g (5S, 6R)-(E,E)-S-(5-Hydroxy-1-hydroxy-1-oxo-eicosa-7,9-dien-11,14-diyn-6-yl)-L-cystein-methylester in einer Mischung aus 10 ml Tetrahydrofuran und 5 ml Wasser, die 0,3 g Lithiumhydroxid enthält, wird während 3 Stunden bei Raumtemperatur stehengelassen. Man versetzt mit Wasser und entfernt die organischen Lösungsmittel im Vakuum. Die erhaltene wässrige Lösung wird an einer mit einem Ionenaustauscher ($H^+$-Form) beladenen Kolonne unter Eluieren mit Wasser und 1N-wässrigem Ammoniak chromatographiert. Das Ammoniak enthaltende Eluat wird gefriergetrocknet und liefert (5S, 6R)-(E,E)-S-(5-Hydroxy-1-hydroxy-1-oxo-eicosa-7,9-dien-11,14-diyn-6-yl)-L-cystein-ammoniumsalz.

Beispiel 17

Durch Hydrierung von (R,S)-(E,E)-3-(1,3-Tetradecadien-5,8-diyn-1-yl)-1-cis-oxiranbuttersäuremethylester in Analogie zu den Angaben im Beispiel 11 erhält man (R,S)-(E,E,Z,Z)-3-(1,3,5,8-Tetradecatetraen-1-yl)-1-cis-oxiranbuttersäuremethylester.

Beispiel 18

Durch Umsetzen von (R,S)-(E,E,Z,Z)-3-(1,3,5,8-tetradecatetraen-1-yl)-1-cis-oxiranbuttersäuremethylester mit L-Cystein-methylester-hydrochlorid in Analogie zu den Angaben im Beispiel 12 erhält man (5S, 6S)-(E,E,Z,Z)-S- (5-Hydroxy-1-methoxy-1-oxo-eicosa-7,9,11,14-tetraen-6-yl)-L-cystein-methylester und (5R, 6R)-(E,E,Z,Z)-S-(5-hydroxy-1-methoxy-1-oxo-eicosa-7,9,11,14-tetraen-6-yl)-L-cystein-methylester. Diese Stoffe werden in Analogie zu den Angaben im Beispiel 12 getrennt.

Beispiel 19

Durch Hydrolyse von (5S, 6S)-(E,E,Z,Z)-S-(5-Hydroxy-1-methoxy-1-oxo-eicosa-7,9,11,14-tetraen-6-yl)-L-cystein-methylester in Analogie zu den Angaben im Beispiel 13 erhält man (5S, 6S)-(E,E,Z,Z)-S-(5-Hydroxy-1-hydroxy-1-oxo-eicosa-7,9,11,14-tetraen-6-yl)-L-cystein-mono-ammoniumsalz.

Beispiel 20

Durch Hydrolyse von (5R, 6R)-(E,E,Z,Z)-S-(5-Hydroxy-1-methoxy-1-oxo-eicosa-7,9,11,14-tetraen-6-yl)-L-cystein-methylester in Analogie zu den Angaben im Beispiel 13 erhält man (5R, 6R)-(E,E,Z,Z)-S-(5-Hydroxy-1-hydroxy-1-oxo-eicosa-7,9,11,14-tetraen-6-yl)-L-cystein-mono-ammoniumsalz.

Beispiel 21

Eine Lösung von 2 g (5S, 6R)-(E,E,Z,Z)-S-(5-Hydroxy-1-hydroxy-1-oxo-eicosa-7,9,11,14-tetraen-6-yl)-L-cystein-dimethylester in 50 ml Methanol wird bei Raumtemperatur mit einer Lösung von 1 g Kaliumhydroxid in 10 ml Wasser versetzt, während 1,5 Stunden bei Raumtemperatur stehengelassen und mit 50 ml Wasser versetzt. Nach Entfernen des Methanols im Vakuum wird die erhaltene wässrige Lösung chromatographiert. Als Adsorptionsmaterial verwendet man einen an Kieselgel gebundenen $C_{18}$-Kohlenwasserstoff (reversed phase). Die Kolonne wird zuerst mit 5–6 Kolonnenvolumen Wasser gewaschen. Das gewünschte Material erhält man durch Eluieren mit Wasser/Methanol (1:4). Nach Entfernen des Methanols im Vakuum und gefriertrocknen der verbleibenden wässrigen Lösung erhält man (5S, 6R)-(E,E,Z,Z)-S-(5-Hydroxy-1-hydroxy-1-oxo-eicosa-7,9,11,14-tetraen-6-yl)-L-cystein-kaliumsalz als leder-farbenen Festkörper.

Beispiel 22

Aus einer Mischung von (5R, 6S)- und (5S, 6R)-(E,E,Z,Z)-S-(5-Hydroxy-1-methoxy-1-oxo-eicosa-8,10,12,15-tetraen-6-yl)-L-cystein-methylester erhält man in Analogie zu den Angaben im Beispiel 13 eine Mischung aus (5R, 6S)- und (5S, 6R)-(E,E,Z,Z)-S-(5-Hydroxy-1-hydroxy-1-oxo-eicosa-7,9,11,14-tetraen-6-yl)-L-cystein-mono-ammoniumsalz.

Experiment 1

Dieses Experiment beschreibt die Isolierung und die Bestimmung der Aktivität von natürlichem SRS-A.

Das SRS-A wurde erhalten, indem man zerkleinerte Lungenstücke von aktiv sensibilisierten Meerschweinchen in vitro mit Eieralbumin anregt. Männliche Tiere (200–250 g) werden 28–45 Tage vor Versuchsbeginn durch intraperitoneale Injektion einer Lösung von 10 mg Antigen (Eieralbumin) in 1 ml 0,9%iger Natriumchloridlösung gegen Eieralbumin sensibilisiert. Nachdem man die Tiere betäubt und ihnen das Blut entnommen hat, entfernt man sofort die Lunge und legt sie in Tyrodes-Lösung (pH 7,4) ein. Die wässrige Tyrodes-Lösung setzt sich wie folgt zusammen: 136,7 mMol/l Natriumchlorid, 2,7 mMol/l Kaliumchlorid, 1,05 mMol/l Magnesiumchlorid, 11,9 mMol/l Natriumbicarbonat, 1,8 mMol/l Calciumchlorid, 0,48 mMol/l Natriumdihydrogenphosphat und 5,5 mMol/l Glucose. Das Lungengewebe wird von Hauptarterien und Blutgefässen befreit, in 1 $mm^3$ grosse Stücke zerkleinert, filtriert und mit Tyrodes-Lösung von Blut freigewaschen. Die zerkleinerte Lunge wird mit Löschpapier getrocknet und in Tyrodes-Lösung suspendiert (150 mg Lungengewebe/ml). Diese Suspension wird während 5 Minuten bei 37° vorinkubiert und mit Eieralbumin (40 mg/ml) versetzt. Nach 10 Minuten wird die SRS-A enthaltende Lösung durch Filtration über Papier vom Lungengewebe befreit.

Die Aktivität dieser das SRS-A enthaltenden Lösung wird nach Orange & Austen, J. Immunol.

10, 105 (1969) wie folgt ermittelt: Ein 1,5 cm grosses Krummdarmsegment aus einem 250–300 g schweren Meerschweinchen wird in einem 10-ml-Organbad, das Tyrodes-Lösung enthält, suspendiert. Dieses Bad enthält $10^{-6}$ Mol Atropinsulfat, um allfällige durch Acetylcholin bewirkte Kontraktionen zu blockieren, und $10^{-6}$ Mol Pyrilamin-maleat, um allfällige durch Histamin induzierte Kontraktionen zu vermeiden. Das Bad wird unter Einleiten eines Gasgemisches, bestehend aus 95% Sauerstoff und 5% Kohlendioxid, bei 37° gehalten. Das obige Segment wird nun mit einem Messgerät verbunden, das die Kontraktionen registriert und aufzeichnet. Die Konzentration von natürlichem SRS-A in obigem Filtrat wird nun ermittelt, indem man die Anzahl Kontraktionen, welche durch Zugabe eines bestimmten Volumens des obigen Filtrates bewirkt werden, mit der Anzahl Kontraktionen, welche durch 5 mg Histamin (in Abwesenheit von Pyrilamin-maleat) hervorgerufen wird, vergleicht. Eine Einheit SRS-A entspricht derjenigen Menge, welche notwendig ist, um die gleiche Anzahl Kontraktionen zu bewirken wie die obige Menge an Histamin. Das im ersten Absatz erhaltene Filtrat enthält 270 Einheiten SRS-A/ml. Nach Standardisierung wird dieses Material in kleinen Aliquoten bei −80° aufbewahrt.

Experiment 2

Es werden die SRS-A-Aktivitäten der folgenden Stoffe ermittelt:

A = (5S, 6R)-(E,E,Z,Z)-S-(5-Hydroxy-1-hydroxy-oxo-eicosa-7,9,11,14-tetraen-6-yl)-L-cystein-mono-ammoniumsalz,

B = (5R, 6S)-(E,E,Z,Z)-S-(5-Hydroxy-1-methoxy-1-oxo-eicosa-7,9,11,14-tetraen-6-yl)-L-cystein-mono-ammoniumsalz,

C = (5S, 6R)-(E,E)-S-(5-Hydroxy-1-hydroxy-1-oxo-eicosa-7,9-dien-11,14-diyn-6-yl)-L-cystein-mono-ammoniumsalz,

D = Mischung aus B und C (1:1),

E = (5S, 6R)-(E,E,Z,Z)-S-(5-Hydroxy-1-oxo-eicosa-7,9,11,14-tetraen-6-yl)-L-cystein-mono-kaliumsalz und

F = (5R, 6S)-(E,E,Z,Z)-S-(5-Hydroxy-1-methoxy-1-oxo-eicosa-7,9,11,14-tetraen-6-yl)-L-cystein-mono-kaliumsalz.

Verschiedene Konzentrationen der obigen Stoffe werden in dem im zweiten Absatz von Experiment 1 beschriebenen biologischen Versuch eingesetzt, um die maximale Anzahl Kontraktionen zu ermitteln, welche diese Stoffe bewirken können. Der in der folgenden Tabelle angegebene $EC_{50}$-Wert ist diejenige Konzentration der Testverbindung, welche 50% der durch natürliches SRS-A bewirkten maximalen Anzahl Kontraktionen bewirkt. Die $EC_{50}$-Werte wurden an mehreren verschiedenen Krummdarm-Präparaten ermittelt. Jeder Wert entspricht dem Durchschnittswert ± Standardabweichung (bestimmt an «n» verschiedenen Krummdarm-Präparaten).

| Testverbindung | $EC_{50}$ in Mol/l | |
|---|---|---|
| A | $5{,}0 \times 10^{-9} \pm 1{,}1$ | n = 3 |
| B | $2{,}8 \times 10^{-8} \pm 0{,}3$ | n = 2 |
| C | $1{,}1 \times 10^{-6} \pm 0{,}1$ | n = 2 |
| D | $4{,}2 \times 10^{-8} \pm 0{,}5$ | n = 5 |
| E | $4{,}3 \times 10^{-9} \pm 0{,}2$ | n = 4 |
| F | $1{,}2 \times 10^{-8} \pm 0{,}2$ | n = 2 |

Experiment 3

In diesem Experiment wird die Aktivität eines bekannten SRS-A-Antagonisten gegen natürliches SRS-A und gegen synthetisches SRS-A-aktives Material ermittelt.

Der in diesem Experiment verwendete bekannte SRS-A-Antagonist ist 7-[3-(4-Acetyl-3-hydroxy-2-propylphenoxy)-2-hydroxypropoxy]-4-oxo-8-propyl-4H-benzopyran-2-carbonsäure (Verbindung G). Das verwendete natürliche SRS-A wurde gemäss den Angaben in Absatz 1 von Experiment 1 erhalten. Die verwendeten synthetischen SRS-A-aktiven Materialien sind die in Experiment 2 mit A und D bezeichneten Stoffe.

Der Versuch wird in Analogie zu den Angaben in Absatz 2 von Experiment 1 durchgeführt. Die Verbindung G wird in verschiedenen Konzentrationen dem Versuchsbad beigegeben, wobei man jeweils ein Gesamtvolumen von 10 ml beibehält, und während 3 Minuten mit dem Gewebe inkubiert. Nach dieser Inkubationszeit versetzt man mit einer Probe an SRS-A-aktivem Material, die derjenigen Menge entspricht, die in Abwesenheit eines Antagonisten 50% der maximal erhältlichen Anzahl Kontraktionen bewirkt. Durch Bildung der jeweiligen Differenz zwischen der Anzahl Kontraktionen in Abwesenheit der Verbindung G und in Anwesenheit von verschiedenen Konzentrationen der Verbindung G lässt sich die Hemmung der durch SRS-A induzierten Anzahl Kontraktionen ermitteln. Nachfolgend sind die $IC_{50}$-Werte angegeben, die diejenige Menge darstellen, welche notwendig ist, um eine Hemmung von 50% zu bewirken. Angegeben wird der jeweilige Durchschnittswert ± Standardabweichung (aus «n» verschiedenen Bestimmungen).

Für die Verbindung G wurde gegen natürliches SRS-A ein $IC_{50}$-Wert von $3{,}7 \times 10^{-8} \pm 0{,}3$ (n = 4) ermittelt. Für die Verbindung G wurde gegen die Verbindung A ein $IC_{50}$-Wert von $3{,}5 \times 10^{-8}$ und $4{,}0 \times 10^{-8}$ (n = 1) ermittelt. Für Verbindung G wurde gegen Verbindung D ein $IC_{50}$-Wert von $4{,}0 \times 10^{-8} \pm 0{,}2$ (n = 2) ermittelt.

Experiment 4

Der im Experiment 3 beschriebene Versuch wird wiederholt, und zwar unter Verwendung von (R,S)-(all E)-3-(1,3-Tetradecadien-5,8-diyn-1-yl)-trans-oxiranbuttersäure-methylester als Antagonist und die Verbindung C als SRS-A-aktive Verbindung. Der ermittelte $IC_{50}$-Wert betrug $1 \times 10^{-5}$ Mol/l (n = 1).

Beispiel A

Es werden in herkömmlicher Weise Tabletten folgender Zusammensetzung hergestellt:

| | mg/Tablette | | | |
|---|---|---|---|---|
| 1. (R,S)-(all E)-3-(1,3-Tetradecadien-5,8-diyn-1-yl)-trans-oxiranbuttersäure-methylester | 10 | 25 | 50 | 100 |
| 2. vorgelatinisierte Stärke | 10 | 15 | 20 | 25 |
| 3. modifizierte Stärke | 10 | 15 | 20 | 25 |
| 4. Lactose | 158 | 177,5 | 187 | 226,5 |
| 5. Talk | 10 | 15 | 20 | 20 |
| 6. Magnesiumstearat | 2 | 2,5 | 3 | 3,5 |
| | 200 | 250 | 300 | 400 |

Verfahren

Die Bestandteile 1–4 werden vermischt, gemahlen und erneut vermischt. Die erhaltene Mischung wird mit Wasser granuliert, über Nacht getrocknet, gemahlen, mit den Bestandteilen 5 und 6 vermischt und verpresst.

Beispiel B

Es werden in herkömmlicher Weise Tabletten folgender Zusammensetzung hergestellt:

| | mg/Tablette | | | |
|---|---|---|---|---|
| 1. (R,S)-(all E)-3-(1,3-Tetradecadien-5,8-diyn-1-yl)-trans-oxiranbuttersäure-methylester | 10 | 25 | 50 | 100 |
| 2. Lactose | 108 | 147,5 | 157 | 345 |
| 3. modifizierte Stärke | 20 | 25 | 30 | 50 |
| 4. Microkristalline Cellulose | 40 | 50 | 60 | 100 |
| 5. Magnesiumstearat | 2 | 2,5 | 3 | 5 |
| | 180 | 250 | 300 | 600 |

Verfahren

Man vermischt die Bestandteile 1–4 in einem geeigneten Mixer, versetzt mit Bestandteil 5, mischt während 5 Minuten und verpresst auf einer geeigneten Presse.

Beispiel C

Es werden Kapseln folgender Zusammensetzung hergestellt:

| | mg/Kapsel | | | |
|---|---|---|---|---|
| 1. (R,S)-(all E)-3-(1,3-Tetradecadien-5,8-diyn-1-yl)-trans-oxiranbuttersäure-methylester | 10 | 25 | 50 | 100 |
| 2. Lactose | 145 | 143 | 168 | 187 |
| 3. Kornstärke | 40 | 40 | 60 | 80 |
| 4. Talk | 3 | 15 | 20 | 30 |
| 5. Magnesiumstearat | 2 | 2 | 2 | 3 |
| | 200 | 225 | 300 | 400 |

Verfahren

Man vermischt die Bestandteile 1–3 in einem geeigneten Mischer, versetzt mit dem Talk und dem Magnesiumstearat, mischt während 5 Minuten und füllt auf einer geeigneten Kapselabfüllmaschine ab.

**Patentansprüche für die Vertragsstaaten: BE, CH, DE, FR, GB, IT, LI, LU, NL, SE**

1. Oxiranbuttersäure-Derivate der allgemeinen Formel

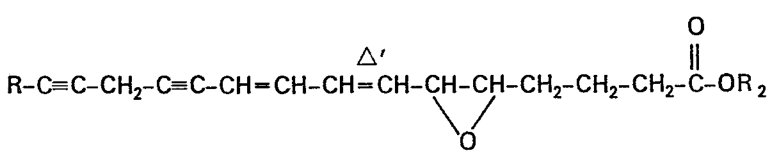

I

worin R die Gruppe $CH_3-CH_2-CH_2-CH_2-CH_2-$ und $R_2$ $(C_{1-7})$-Alkyl, Aryl oder Aryl-$(C_{1-7})$-Alkyl bedeuten, das Symbol $\triangle'$ die trans-Konfiguration der Doppelbindung anzeigt und Aryl gegebenenfalls durch $(C_{1-7})$-Alkyl oder Nitro substituiertes Phenyl, Naphthyl oder Anthryl bedeutet.

2. Verbindungen gemäss Anspruch 1, dadurch gekennzeichnet, dass $R_2$ Methyl, Äthyl, Phenyl oder Benzyl bedeutet.

3. Verbindungen gemäss Anspruch 1 oder 2, dadurch gekennzeichnet, dass diese Verbindungen bezüglich des Oxiranringes die trans-Konfiguration aufweisen.

4. (R,S)-(all E)-3-(1,3-Tetradecadien-5,8-diyn-1-yl)-1-trans-oxiranbuttersäure-methylester.

5. Verbindungen gemäss einem der Ansprüche 1–4 als pharmazeutische Wirkstoffe.

6. Verbindungen gemäss einem der Ansprüche 1–4 als anti-allergisch wirksame Stoffe.

7. Verfahren zur Herstellung von Verbindungen gemäss einem der Ansprüche 1–4, dadurch gekennzeichnet, dass man eine Verbindung der allgemeinen Formel

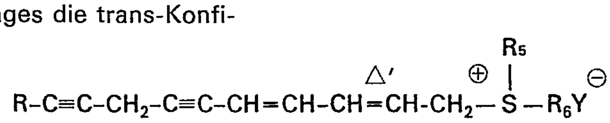

II

worin Y Halogen bedeutet und $R_5$ und $R_6$ unabhängig voneinander Methyl oder Äthyl bedeuten, oder zusammen mit dem Schwefelatom einen gesättigten 4- bis 6gliedrigen heterocyclischen Ring bedeuten, wobei das erwähnte Schwefelatom das einzige Heteroatom ist, und $\triangle'$ die in Anspruch 1 angegebene Bedeutung besitzt, in Gegenwart einer starken Base mit einer Verbindung der allgemeinen Formel

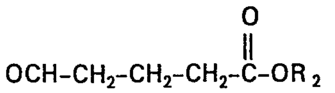
III

worin $R_2$ die in Anspruch 1 angegebene Bedeutung besitzt, umsetzt, und erwünschtenfalls den erhaltenen Stoff bezüglich des Oxiranringes in die cis- oder trans-Isomeren auftrennt.

8. Arzneimittel, enthaltend eine Verbindung gemäss einem der Ansprüche 1-4.

9. Anti-allergisches Mittel, enthaltend eine Verbindung gemäss einem der Ansprüche 1-4.

**Patentansprüche für den Vertragsstaat AT**

1. Verfahren zur Herstellung von Oxiranbuttersäure-Derivaten der allgemeinen Formel

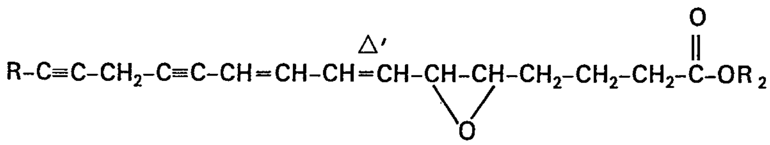
I

worin R die Gruppe $CH_3-CH_2-CH_2-CH_2-CH_2-$ und $R_2$ $(C_{1-7})$-Alkyl, Aryl oder Aryl-$(C_{1-7})$-Alkyl bedeuten, das Symbol △′ die trans-Konfiguration der Doppelbindung anzeigt und Aryl gegebenenfalls durch $(C_{1-7})$-Alkyl oder Nitro substituiertes Phenyl, Naphthyl oder Anthryl bedeutet, dadurch gekennzeichnet, dass man eine Verbindung der allgemeinen Formel

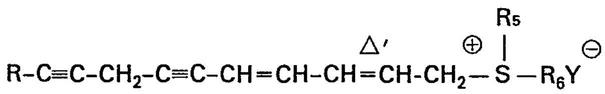
II

worin Y Halogen bedeutet und $R_5$ und $R_6$ unabhängig voneinander Methyl oder Äthyl bedeuten, oder zusammen mit dem Schwefelatom einen gesättigten 4- bis 6gliedrigen heterocyclischen Ring bedeuten, wobei das erwähnte Schwefelatom das einzige Heteroatom ist, und △′ obige Bedeutung besitzt, in Gegenwart einer starken Base mit einer Verbindung der allgemeinen Formel

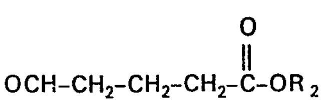
III

worin $R_2$ obige Bedeutung besitzt, umsetzt, und erwünschtenfalls den erhaltenen Stoff bezüglich des Oxiranringes in die cis- und trans-Isomeren auftrennt.

2. Verfahren gemäss Anspruch 1, dadurch gekennzeichnet, dass man eine Verbindung der in Anspruch 1 definierten allgemeinen Formel II mit einer Verbindung der in Anspruch 1 definierten allgemeinen Formel III, worin $R_2$ Methyl, Äthyl, Phenyl oder Benzyl bedeutet, umsetzt.

3. Verfahren gemäss Anspruch 1 oder 2, dadurch gekennzeichnet, dass man aus einem Gemisch der bezüglich des Oxiranringes cis- und trans-Isomeren das trans-Isomere isoliert.

4. Verfahren gemäss Anspruch 1, zur Herstellung von (R,S)-(all E)-3-(1,3-Tetradecadien-5,8-diyn-1-yl)-1-trans-oxiranbuttersäure-methylester, dadurch gekennzeichnet, dass man eine Verbindung der in Anspruch 1 definierten allgemeinen Formel II mit 4-Formyl-buttersäure-methylester umsetzt und aus dem erhaltenen Gemisch der bezüglich des Oxiranringes cis- und trans-Isomeren das trans-Isomere isoliert.

**Claims for the Contracting States: BE, CH, DE, FR, GB, IT, LI, LU, NL, SE.**

1. Oxiranebutyric acid derivatives of the general formula

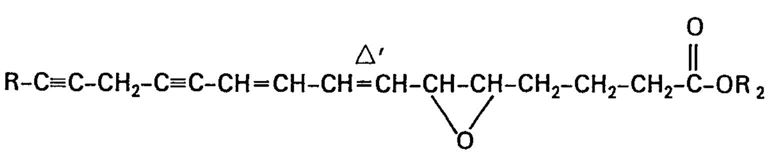
I

wherein R signifies the group $CH_3-CH_2-CH_2-CH_2-CH_2-$ and $R_2$ signifies $(C_{1-7})$-alkyl, aryl or aryl-$(C_{1-7})$-alkyl, the symbol △′ indicates the trans configuration of the double bond and aryl signifies phenyl, naphthyl or anthryl optionally substituted by $(C_{1-7})$-alkyl or nitro.

2. Compounds according to claim 1, characterized in that $R_2$ signifies methyl, ethyl, phenyl or benzyl.

3. Compounds according to claim 1 or 2, characterized in that these compounds have the trans configuration with respect to the oxirane ring.

4. (R,S)-(All E)-3-(1,3-tetradecadien-5,8-diyn-1-yl)-1-trans-oxiranebutyric acid methyl ester.

5. Compounds according to any one of claims 1-4 as pharmaceutically active substances.

6. Compounds according to any one of claims 1-4 as anti-allergic active substances.

7. A process for the manufacture of compounds according to any one of claims 1-4, characterized by reacting a compound of the general formula

$$R\text{-}C{\equiv}C\text{-}CH_2\text{-}C{\equiv}C\text{-}CH{=}CH\text{-}\overset{\triangle'}{CH{=}CH}\text{-}CH_2\text{-}\overset{\oplus\ R_5}{\overset{|}{S}}\text{-}R_6\overset{\ominus}{Y} \qquad II$$

wherein Y signifies halogen and $R_5$ and $R_6$ independently of each other signify methyl or ethyl or together with the sulphur atom signify a saturated 4- to 6-membered heterocyclic ring, whereby the mentioned sulphur atom is the sole hetero atom, and △′ has the significance given in claim 1, in the presence of a strong base with a compound of the general formula

$$OCH\text{-}CH_2\text{-}CH_2\text{-}CH_2\text{-}\overset{\overset{O}{\|}}{C}\text{-}OR_2 \qquad III$$

wherein $R_2$ has the significance given in claim 1, and, if desired, separating the substance obtained into the cis or trans isomer with respect to the oxirane ring.

8. A medicament containing a compound according to any one of claims 1–4.

9. An anti-allergic medicament containing a compound according to any one of claims 1–4.

**Claims for the Contracting State: AT.**

1. A process for the manufacture of oxiranebutyric acid derivatives of the general formula

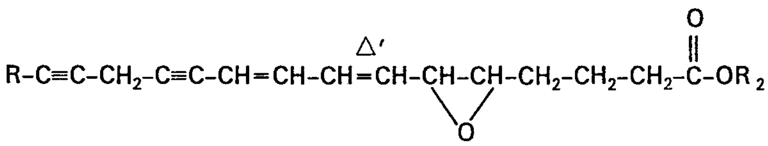

I

wherein R signifies the group $CH_3\text{-}CH_2\text{-}CH_2\text{-}CH_2\text{-}CH_2\text{-}$ and $R_2$ signifies $(C_{1-7})$-alkyl, aryl or aryl-$(C_{1-7})$-alkyl, the symbol △′ indicates the trans configuration of the double bond and aryl signifies phenyl, naphthyl or anthryl optionally substituted by $(C_{1-7})$-alkyl or nitro, characterized by reacting a compound of the general formula

$$R\text{-}C{\equiv}C\text{-}CH_2\text{-}C{\equiv}C\text{-}CH{=}CH\text{-}\overset{\triangle'}{CH{=}CH}\text{-}CH_2\text{-}\overset{\oplus\ R_5}{\overset{|}{S}}\text{-}R_6\overset{\ominus}{Y} \qquad II$$

wherein Y signifies halogen and $R_5$ and $R_6$ independently of each other signify methyl or ethyl or together with the sulphur atom signify a saturated 4-to 6-membered heterocyclic ring, whereby the mentioned sulphur atom is the sole hetero atom, and △′ has the above significance, in the presence of a strong base with a compound of the general formula

$$OCH\text{-}CH_2\text{-}CH_2\text{-}CH_2\text{-}\overset{\overset{O}{\|}}{C}\text{-}OR_2 \qquad III$$

wherein $R_2$ has the above significance, and, if desired, separating the substance obtained into the cis and trans isomers with respect to the oxirane ring.

2. A process according to claim 1, characterized in that a compound of general formula II defined in claim 1 is reacted with a compound of general formula III defined in claim 1 wherein $R_2$ signifies methyl, ethyl, phenyl or benzyl.

3. A process according to claim 1 or 2, characterized in that the trans isomer is isolated from a mixture of the cis and trans isomers with respect to the oxirane ring.

4. A process according to claim 1 for the manufacture of (R,S)-(all E)-3-(1,3-tetradecadien-5,8-diyn-1-yl)-1-trans-oxiranebutyric acid methyl ester, characterized by reacting a compound of general formula II defined in claim 1 with 4-formyl-butyric acid methyl ester and isolating the trans isomer from the resulting mixture of cis and trans isomers with respect to the oxirane ring.

**Revendications pour les Etats Contractants: BE, CH, DE, FR, GB, IT, LI, LU, NL, SE**

1. Dérivés d'acide oxiranebutyrique de formule générale

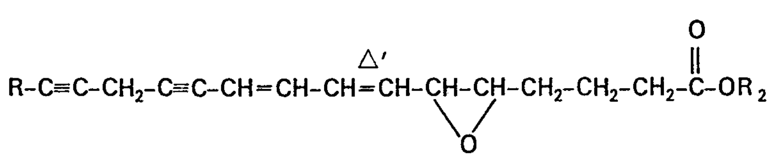

I

où R représente le groupe $CH_3\text{-}CH_2\text{-}CH_2\text{-}CH_2\text{-}CH_2\text{-}$ et $R_2$ représente un alcoyle en $C_1$ à $C_7$, un aryle ou un aryl-alcoyle en $C_1$ à $C_7$, le symbole △′ montre la configuration trans de la double liaison et aryle représente un phényle, un naphtyle ou un anthryle éventuellement substitué par un alcoyle en $C_1$ à $C_7$ ou un nitro.

2. Composés selon la revendication 1, caracté-

risés en ce que $R_2$ représente un méthyle, un éthyle, un phényle ou un benzyle.

3. Composés selon l'une des revendications 1 ou 2, caractérisés en ce que ces composés présentent par rapport au noyau oxirane la configuration trans.

4. Ester méthylique de l'acide (R,S)-(tout E)-3-(1,3-tétradécadiène-5,8-diyn-1-yl)-1-trans-oxiranebutyrique.

5. Composés selon l'une des revendications 1-4 comme substances actives pharmaceutiques.

6. Composés selon l'une des revendications 1-4 comme substances actives anti-allergiques.

7. Procédé de préparation de composés selon l'une des revendications 1-4, caractérisé en ce qu'on fait réagir un composé de formule générale

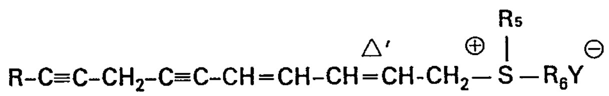 II

où Y représente un halogène et $R_5$ et $R_6$ représentent indépendamment l'un de l'autre un méthyle ou un éthyle, ou représentent ensemble avec l'atome de soufre un noyau hétérocyclique à 4 à 6 chaînons saturé, où l'atome de soufre mentionné est le seul hétéroatome, et △' a la signification donnée dans la revendication 1, en présence d'une base forte avec un composé de formule générale

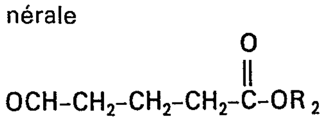 III

où $R_2$ a la signification donnée dans la revendication 1, et si on le désire en ce qu'on dédouble le corps obtenu en les isomères cis ou trans par rapport au noyau oxirane.

8. Médicament contenant un composé selon l'une des revendications 1-4.

9. Agent anti-allergique contenant un composé selon l'une des revendications 1-4.

**Revendications pour l'Etat Contractant: AT**

1. Procédé de préparation de dérivés d'acide oxiranebutyrique de formule générale

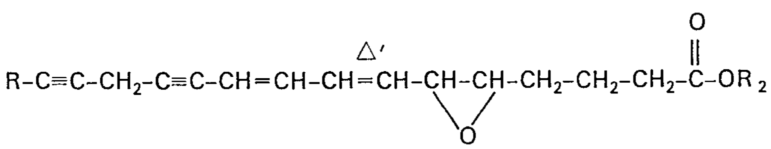 I

où R représente le groupe $CH_3$-$CH_2$-$CH_2$-$CH_2$-$CH_2$- et $R_2$ représente un alcoyle en $C_1$ à $C_7$, un aryle ou un aryl-alcoyle en $C_1$ à $C_7$, le symbole △' montre la configuration trans de la double liaison et aryle représente un phényle, un naphtyle ou un anthryle éventuellement substitué par un alcoyle en $C_1$ à $C_7$ ou un nitro, caractérisé en ce qu'on fait réagir un composé de formule générale

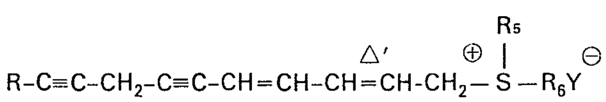 II

où Y représente un halogène et $R_5$ et $R_6$ représentent indépendamment l'un de l'autre un méthyle ou un éthyle, ou représentent ensemble avec l'atome de soufre un noyau hétérocyclique à 4 à 6 chaînons saturé, où l'atome de soufre mentionné est le seul hétéroatome, et △' a la signification donnée ci-dessus, en présence d'une base forte avec un composé de formule générale

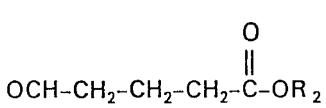 III

où $R_2$ a la signification donnée ci-dessus, et si on le désire en ce qu'on dédouble le corps obtenu en les isomères cis et trans par rapport au noyau oxirane.

2. Procédé selon la revendication 1, caractérisé en ce qu'on fait réagir un composé de formule générale II définie dans la revendication 1 avec un composé de formule générale III défini dans la revendication 1, où $R_2$ représente un méthyle, un éthyle, un phényle ou un benzyle.

3. Procédé selon l'une des revendications 1 ou 2, caractérisé en ce qu'on isole l'isomère trans à partir d'un mélange d'isomères cis et trans par rapport au noyau oxirane.

4. Procédé selon la revendication 1 de préparation d'ester méthylique de l'acide (R,S)-(tout E)-3-(1,3-tétradécadiène-5,8-diyn-1-yl)-1-trans-oxiranebutyrique, caractérisé en ce qu'on fait réagir un composé de formule générale II défini dans la revendication 1 avec l'ester méthylique de l'acide 4-formylbutyrique et en ce qu'on isole l'isomère trans à partir du mélange obtenu des isomères cis et trans par rapport au noyau oxirane.